# EUROPÄISCHE PATENTSCHRIFT

(11) **EP 1 304 993 B1**
(45) Veröffentlichungstag und Bekanntmachung des Hinweises auf die Patenterteilung: **11.10.2006**
(21) Anmeldenummer: 01955362.7
(22) Anmeldetag: 19.07.2001
(51) Int. Cl.: A61K 8/46, A61Q 5/04

(54) **DAUERWELLVERFAHREN**
PERMING METHOD
PROCEDE POUR L'ONDULATION PERMANENTE DES CHEVEUX

(30) Priorität: 28.07.2000 DE 10036749
(43) Veröffentlichungstag der Anmeldung: 02.05.2003
(73) Patentinhaber: Henkel Kommanditgesellschaft auf Aktien, 40589 Düsseldorf (DE)
(72) Erfinder: NEUBÜSER, Inge, 22589 Hamburg (DE); MÜLLER, Burkhard, 21075 Hamburg (DE)
(86) Internationale Anmeldenummer: PCT/EP2001/008361
(87) Internationale Veröffentlichungsnummer: WO 2002/009659

(56) Entgegenhaltungen:
- EP-A- 0 167 866
- EP-A- 0 492 119
- DE-A- 3 610 394
- GB-A- 671 844
- US-A- 4 038 995

## Beschreibung

Die Erfindung betrifft ein Verfahren zur dauerhaften Verformung von Keratinfasern, insbesondere von menschlichen Haaren durch Sulfitolyse des Haarkeratins und anschließende Hydrolyse mit Wasser oder einer wäßrigen Lösung eines Puffersalzes.

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann mit einer Oxidationsmittelzubereitung behandelt, gespült und von den Verformungshilfsmitteln (Wicklern, Papilloten) befreit.

Wenn als keratinreduzierende Komponente ein Merkaptan, z.B. ein Natriumthioglycolat verwendet wird, spaltet dieses einen Teil der Disulfid-Brücken des Keratin-Moleküls zu -SH-Gruppen, so daß es zu einer Erweichung der Keratinfaser kommt. Bei der späteren oxidativen Fixierung werden erneut Disulfid-Brücken im Haarkeratin geknüpft, so daß das Keratingefüge in der vorgegebenen Verformung fixiert wird. Die Verwendung von Merkaptanen zur Haarverformung ist aber mit erheblichen Nachteilen verbunden.

So weist das Haar nach der Behandlung oft einen unangenehmen Merkaptangeruch auf, der manchmal noch nach Tagen wahrnehmbar ist. Auch kann der hohe pH-Wert der üblichen Merkaptocarbonsäuresalz-Lösungen die Haarstruktur schädigen. Die Verwendung von Merkaptocarbonsäureestem kann schließlich allergische Reaktionen der Haut verursachen.

Es wurde daher schon oft vorgeschlagen, zur Haarverformung anstelle der Merkaptane Sulfit zu verwenden. Durch Hydrogensulfit-Lösungen werden Disulfid-Brücken des Keratins nach der Gleichung

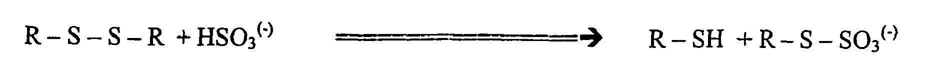

gespalten und auf diese Weise eine Erweichung der Keratinfaser erreicht. Leider kann man das Haarkeratin nach der Sulfitolyse nicht mehr so gut wie nach der Verformung mit Merkaptanen durch Behandlung mit Oxidationsmitteln fixieren. Die mit sulfithaltigen Verformungsmittel erzielte Haarverformung weist daher keine befriedigende Haltbarkeit auf. Es wurden daher zahlreiche Vorschläge gemacht, wie man das durch Sulfitolyse erweichte Keratingefüge nach der Verformung wieder regenerieren und festigen kann.

Aus GB 552 286 war z.B. ein Dauerwellverfahren bekannt, bei dem die Keratinfaser mit einer Hydrogensulfit-Lösung behandelt, getrocknet, mechanisch verformt und schließlich mit einer wäßrigen, stark alkalischen Zubereitung mit einem pH-Wert von 10-12 nachbehandelt wird.

Aus US 2,817,342 war ein ähnliches Verfahren bekannt, bei welchem aber die Nachbehandlung mit einer Zubereitung erfolgt, die Harnstoff und Urease enthält, wobei sich nach und nach durch den Harnstoffabbau soviel Ammoniak bildet, daß der pH-Wert auf 8-9 ansteigt.

Aus EP 0 257 256 B1 war ein Dauerwellverfahren bekannt, bei dem ein Gemisch aus Sulfit und Merkaptan zur Anwendung kommt. Es findet aber bei diesem Verfahren keine Sulfitolyse des Keratins statt, vielmehr katalysiert das Sulfit die Bildung gemischter Disulfide und freier SH-Gruppen im Keratin. Bei diesem Verfahren wird das Haar durch Nachbehandlung mit einer oxidationsmittelfreien, wäßrigen Lösung mit einem pH-Wert von 7-12, bevorzugt von 8,7-12 temporär fixiert. Zur dauerhaften Fixierung wird eine zusätzliche oxidative Behandlung vorgeschlagen.

Die vorgenannten Verfahren weisen immer noch die bekannten Nachteile des Standes der Technik auf, insbesondere ist die Nachbehandlung mit einer stark alkalischen Zubereitung problematisch für die Struktur des Haars und für die Kopfhaut.

Es bestand daher nach wie vor ein Bedürfnis, eine schonende Haarverformung unter schwach sauren bis neutralen Bedingungen zu erreichen und unter ebenfalls neutralen bis schwach basischen Bedingungen zu fixieren. Der vorliegenden Erfindung liegt die Beobachtung zugrunde, daß durch ein gründliches Nachbehandeln des durch Sulfitolyse weichgemachten und verformten Keratins mit Wasser oder mit einer wäßrigen, von Oxidationsmitteln freien Lösung eines Puffersalzes mit einem pH-Wert von 6-9 eine weitgehende Fixierung der Verformung ohne nachhaltige Wirkungen für die Struktur des Haars oder der Zustand der Kopfhaut erreicht werden kann.

Gegenstand der Erfindung ist also ein Verfahren zur dauerhaften Verformung von Keratinfasern, bei welchen man die Fasern vor und/oder nach einer mechanischen Verformung einer Sulfitolyse durch Behandlung mit einer wäßrigen Lösung eines Sulfit-Salzes mit einem pH-Wert von 5-8 unterwirft, und die Keratinfasern nach der Sulfitolyse über einen Zeitraum von wenigstens 10 Minuten mit Wasser oder einer wäßrigen Zubereitung eines Puffersalzes mit einem pH-Wert von 6-9 nachbehandelt.

Als Keratinfasern können prinzipiell alle tierischen Haare, z.B. Wolle, Roßhaar, Angorahaar, Pelze, Federn und daraus gefertigte Produkte oder Textilien eingesetzt werden. Bevorzugt eignet sich das erfindungsgemäße Verfahren aber für die Verformung menschlicher Haare und daraus gefertigter Perücken. Wegen der schonenden Verfahrensbedingungen ist es besonders geeignet zur Verformung des Haars am lebenden Körper, also z.B. zur Erzeugung von Dauerwellen oder zur Glättung von Kraushaar.

Die für die erste Stufe des erfindungsgemäßen Verfahrens zur Keratinerweichung (Sulfitolyse) eingesetzte Lösung enthält bevorzugt ein Hydrogensulfit, z.B. als Alkali-, Magnesium-, Ammonium- oder Alkanolammonium-Salz auf Basis eines C₂-C₄-Mono-, Di- oder Trialkanolamins. Ammoniumhydrogensulfit ist ein besonders bevorzugtes Hydrogensulfit, das insbesondere in einer Menge von 5-15 Gew.-%, bezogen auf die gesamte Lösung, eingesetzt wird. Der pH-Wert dieser Lösung wird auf einen Wert im Neutralbereich zwischen pH=5 und 8, bevorzugt auf pH=6-7 eingestellt. Monoethanolamin ist ein besonders bevorzugtes Alkanolamin, das insbesondere in einer Menge von 1-5 Gew.-% bezogen auf die gesamte Lösung, eingesetzt wird. Dazu wird die schwach saure Hydrogensulfit-Lösung durch eine schwache Base, z.B. ein Alkanolamin eingestellt. In einer bevorzugten Ausführung enthält die Lösung zur Durchführung der Sulfitolyse 5-15 Gew.-% Ammoniumhydrogensulfit und 1-5 Gew.-% Monoethanolamin.

Darüberhinaus kann die Hydrogensulfit-Lösung weitere Komponenten enthalten, die die Wirkung auf das Keratin fördern. Solche Komponenten sind z.B. Quellmittel wie z.B. niedere Alkohole und wasserlösliche Glycole oder Polyole wie z.B. Glycerin, 1,2-Propylenglycol oder Sorbit und Harnstoff oder Harnstoffderivate wie z.B. Allantoin und Guanidin sowie Imidazol und dessen Derivate. Bevorzugte weitere Komponenten sind 1,2-Propylenglycol, insbesondere in einer Menge von 5-20 Gew.-%, sowie Harnstoff, insbesondere in einer Menge von 1-10 Gew.-%. Die Mengenangaben beziehen sich jeweils auf die gesamte Lösung. In einer bevorzugten Ausführung enthält die Lösung zur Durchführung der Sulfitolyse 5-20 Gew.-% 1,2-Propylenglycol und/oder 1-10 Gew.-% Harnstoff.

Weiterhin kann die Hydrogensulfit-Lösung oberflächenaktive Stoffe, insbesondere solche aus der Gruppe der anionischen, amphoteren, zwitterionischen und nichtionischen Tenside enthalten. Diese Tenside haben die Aufgabe, die Benetzung der Keratinoberfläche durch die Behandlungslösung zu fördern, vor allem aber die Duftstoffe zu solubilisieren oder stabil zu emulgieren. In einer bevorzugten Ausführung enthält die Lösung zur Durchführung der Sulfitolyse daher nichtionogene Tenside, die ein gutes Emulgier- oder Solublilisierungsvermögen für etherische Öle und Duftstoffe haben. Solche nichtionogenen Tenside sind beispielsweise
- Anlagerungsprodukte von 5-30 Mol Ethylenoxid und 0 bis 5 Mol Propylenoxid an lineare Fettalkohole und 1,2-Alkandiole mit 12-22 C-Atomen, an Fettsäuren mit 12-22 C-Atomen, an Fettsäuremono- und Diglyceride und an Sorbitan-Fettsäureester von C₁₂-C₂₂-Fettsäuren,
- Anlagerungsprodukte von 20-60 Mol Ethylenoxid an Fettsäuretriglyceride, insbesondere an Rizinusöl oder gehärtetes Rizinusöl,
- C₈-C₁₆-Alkyl-(oligo)-glycoside und deren Ethylenoxidanlagerungsprodukte.

Darüberhinaus kann die Hydrogensulfit-Lösung die in solchen Zubereitungen üblichen Hilfs- und Zusatzmittel enthalten. Solche Komponenten sind z.B:
- Duftstoffe (Parfümöle)
- Verdickungsmittel wie z.B. Pflanzen-Gumme, Xanthan-Gum, Alginate, Cellulose- und Stärkeether,
- Proteinhydrolysate
- wasserlösliche Polymere festigender Wirkung, z.B. Polyvinylpyrrolidon, Vinylacetat/Crotonsäure-Copolymere,
- wasserlösliche kationische Polymere mit antistatischer und avivierender Wirkung, z.B. quaternierte Cellulose- oder Guarether, Dimethyl-dialkylammonium-Polymere, Vinylpyrrolidon-Imidazoliniummethochlorid-Copolymere, Trimethylamonioethylmethacrylat-Vinylpyrrolidon-Copolymere,
- Antischuppenwirkstoffe wie z.B. Picrotone Olamine, Zink Omadine,
- Vitamine wie z.B. Tocopherolacetat, Pyridoxin, Panthenol,
- Wirkstoffe wie Allantoin, Pyrrolidoncarbonsäuren, Pflanzenextrakte ,
- Strukturanten wie z.B. Glucose oder Maleinsäure
- pH-Einstell- und Puffermittel wie z.B. Citronensäure/Natriumcitrat, Ammoniumcarbonat, Guanidincarbonat, Phosphate,
- Komplexbildner wie EDTA, NTA, Organophosphonsäuren
- Lichtschutzmittel (UV-Absorber)
- Öl-, Fett- und Wachskomponenten oder Silikone, bevorzugt in emulgierter Form,
- Farbstoffe, Trübungs- und Perlglanzmittel sowie
- gegebenenfalls Aerosol-Treibgase

Die Behandlung der Keratinfasern mit der Lösung des Hydrogensulfit-Salzes wird bei Temperaturen von 15-50 °C über einen Zeitraum von 5-60 Minuten, bevorzugt bei Temperaturen von 20-40°C innerhalb von 10-40 Minuten durchgeführt. Zum Dauerwellen der Haare wird die Lösung bevorzugt auf das auf Wicklern aufgerollte Haar aufgetragen und gegebenenfalls unter Zufuhr von Wärme und unter Verwendung einer Abdeckung einwirken gelassen.

Danach werden die verformten Keratinfasern gut mit Wasser gespült und gegebenenfalls oxidativ fixiert und erneut gespült. Dann erfolgt die erfindungsgemäße Nachbehandlung mit Wasser oder einer wäßrigen Lösung eines Puffersalzes. Im einfachsten Falle kann diese Nachbehandlung in einem längerdauernden Spülen mit Wasser mit einem pH-Wert im Bereich von 6-9 , bevorzugt von 6-8 und bevorzugt mit einer Temperatur von 20-40 °C bestehen. Die Nachbehandlung kann aber auch eine Wäsche mit einem pH-neutralen Shampoo und anschließendes Spülen mit Wasser einschließen.

Wenn es sich bei den verformten Keratinfasern um Textilien aus Wolle oder anderen Tierhaaren handelt, kann die Nachbehandlung in der Weise erfolgen, daß man die Fasern in Wasser einweicht und gegebenenfalls leicht darin bewegt. Im Rahmen einer Dauerwellbehandlung kann man das gewickelte Haar über einen Zeitraum von wenigstens 10 Minuten mit Wasser der vorgenannten Temperatur spülen, indem man warmes Wasser über das eingerollte Haar fließen läßt. Man kann aber auch das mit Wasser oder einer wäßrigen Nachbehandlungszubereitung benetzte Haar abdecken, z.B. mit einer wasserundurchlässigen Haube oder einem Turban und gegebenenfalls unter Wärmeeinwirkung, z.B. unter einer Trockenhaube, das Wasser oder die Zubereitung auf das Haar einwirken lassen. Die Dauer der Nachbehandlung sollte wenigstens 10 Minuten, besser aber mehr, z.B. 20 bis 60 Minuten betragen. Erst danach wird das Haar getrocknet und von den Haarwicklern befreit.

Wenn man für die Nachbehandlung eine geeignete kosmetische Zubereitung verwendet, die ein Puffersalz-Gemisch mit einem pH-Wert von 6 bis 9 enthält, kann man als Puffersalz z.B. Gemische von primären, sekundären und tertiären Alkaliphosphaten, Gemische aus Citronensäure und Alkalicitraten, Gemische aus Milchsäure und Alkalilactaten, Guanidinglycolat, Alkanolammoniumbenzoat und andere Salze schwacher Säuren oder schwacher Basen in einer Menge von 1-10 Gew.-% bezogen auf die wäßrige Zubereitung einsetzen.

In einer bevorzugten Ausführung der Erfindung wird vor der Nachbehandlung eine oxidative Fixierung durchgeführt. Für diesen Zweck kann man wäßrige Zubereitungen der dafür üblichen Oxidationsmittel wie z.B. Natriumbromat, Kaliumbromat oder Wasserstoff- peroxid und der zur Stabilisierung wäßriger Wasserstoffperoxidzubereitungen üblichen Stabilisatoren verwenden. Der pH-Wert solcher wäßriger H₂O₂-Zubereitungen, die üblicherweise etwa 0,5 bis 3,0 Gew.-% H₂O₂ enthalten, liegt bevorzugt bei 2 bis 6. Fixiermittel auf Bromat-Basis enthalten die Bromate üblicherweise in Konzentrationen von 1 bis 10 Gew.-% und der pH-Wert der Lösungen wird auf 4 bis 7 eingestellt.
Oft werden die erfindungsgemäßen Fixiermittel als Feststoffe formuliert. Sie enthalten das Oxidationsmittel dann in Form eines Festkörpers, z.B. Natriumperborat. Erst kurz vor der Anwendung werden diese Mittel dann mit Wasser versetzt.
Ebenfalls möglich und bevorzugt ist, das Oxidationsmittel als 2-Komponenten-System zu formulieren. Die beiden Komponenten, von denen die eine bevorzugt eine schwach saure Wasserstoffperoxidlösung oder eine wäßrige Lösung eines anderen Oxidationsmittels ist und die andere die übrigen Bestandteile und eine Base enthält, werden ebenfalls erst kurz vor der Anwendung zu einer gebrauchsfertigen Fixierlösung mit einem pH-Wert von 7-9 vermischt (vergl. US 5,775,342).

Anstelle oder zusätzlich zu den im Zusammenhang mit dem Wellmittel genannten Tensiden können die Fixiermittel als oberflächenaktive Verbindungen auch kationische Tenside enthalten.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind die sogenannten Esterquats wie die unter dem Warenzeichen Stepantex^{R} vertriebenen Methyl-hydroxyalkyl-dialkoyloxyalkylammoniummethosulfate.

Darüberhinaus können die Fixiermittel alle bereits für die Wellmittel genannten Hilfs- und Zusatzmittel enthalten.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, CO₂, Luft, N₂O, Dimethylether, Fluorchlorkohlenwasserstofftreibmitteln oder Gemischen davon in Aerosolbehältern mit Schaumventil abgefüllt werden.
Die folgenden Beispiele sollen den Gegenstand der Erfindung näher erläutern:

### Beispiele

| **1. Well-Lotion** | **1.1** | **1.2** | **1.3** |
|---|---|---|---|
| Ammoniumbisulfit (70%-ig) | 14,3 Gew.-% | 14,3 Gew.-% | 14,3 Gew.-% |
| Monoethanolamin | 3,67 | --- | 2,8 |
| 1,2-Propylenglycol | 10,00 | 5,0 | 5,0 |
| Harnstoff | 5,00 | --- | --- |
| Imidazol | --- | 5,0 | 5,0 |
| Cremophor® RH40 (1) | 1,00 | --- | 1,25 |
| Tween® 20 (7) | --- | 2,0 | 1,25 |
| Eumulgin® L (2) | 1,00 | --- | --- |
| Merquat® 280 (6) | --- | 0,2 | --- |
| Citronensäure | --- | --- | 3,0 |
| Ascorbinsäure | --- | 0,1 | --- |
| Parfüm | 0,3 | --- | 0,5 |
| Isopropylalkohol | --- | 1,0 | 1,0 |
| Wasser | 64,73 | 72,4 | 65,9 |
| pH-Wert | 6,7 | 6,8 | 6,9 |

| **2.1. Fixierlösung (pH = 3,8)** | **Gew.-%** |
|---|---|
| Wasserstoffperoxid (50%-ig) | 5,2 |
| Ammoniak (25%-ig) | 0,75 |
| Dipicolinsäure | 0,10 |
| 1-Hydroxyethan-1,1-diphosphonsäure | 1,80 |
| Dehyquart® E (3) | 1,00 |
| Euperlan® PK 771 (4) | 10,00 |
| Parfüm | 0,50 |
| Wasser | 80,65 |

| **2.2 Fixierlösung** | **Teillösung A** | **Teillösung B** |
|---|---|---|
| Wasserstoffperoxid (50%-ig) | --- | 4,20 Gew.-% |
| Triethanolamin | 10,7 Gew.-% | ----- |
| Croquat® WKP (5) | 6,5 Gew.-% | ----- |
| Dehyquart® E (3) | 0,9 Gew.% | ----- |
| Euperlan® PK771 (4) | 9,0 Gew.% | ----- |
| Methylparaben | 0,2 Gew.-% | ----- |
| Phosphorsäure | ----- | 0,01 Gew.-% |
| NaH₂PO₄ | ----- | 0,42 Gew.-% |
| Parfüm | 0,5 Gew.-% | ----- |
| Wasser | 72,2 Gew.-% | 95,37 Gew.-% |

Vor Gebrauch wurden 17 Gewichtsteile (A) mit 83 Gewichtsteilen (B) vermischt, um eine gebrauchsfertige Fixierlösung mit einem pH-Wert von 8,5 zu erhalten.

Es wurden die folgendne Handelsprodukte verwendet:

| | |
|---|---|
| (1) Cremophor® RH40: | hydr.Rizinusöloxethylat (40 Mol EO) |
| (BASF) | |
| | |
| (2) Eumulgin® L: | PPG-1-PEG-9-Lauryl Glycol Ether |
| (Cognis Deutschland) | |
| | |
| (3) Dehyquart® E: hydroxy- | N,N-Dimethyl-N-(2-hydroxyethyl)-N-(2-hexadecyl)-ammoniumchlorid (25%-ig) |
| (Cognis Deutschland) | |
| | |
| (4) Euperlan® PK771: | Perlglanzkonzentrat auf Basis von Ethylenglycoldistearat, Kokosfettsäuremonoethanolamid, Laurylethersulfat, Laurylpolyglycolether (45 Gew.-% Feststoff). |
| (Cognis Deutschland) | |
| (5) Croquat® WKP: | Cocodimonium Hydroxypropyl Hydrolyzed Keratin, (32 Gew.-% Feststoff) |
| (Croda GmbH) | |
| | |
| (6) Merquat® 280: | Dimethyldiallylammonium-Acrylsäure Copolymer (Polyquaternium 22) |
| | (40 %-ig in Wasser) |
| | |
| (7) Tween® 20: | Polyoxyethylensorbitan-monolaurat |
| | (Polysorbate 20) |

### Versuchsdurchführung:

Die Haare von 5 Testpersonen wurden zunächst mechanisch verformt (auf Lockenwickler gewickelt) und bei 40°C mit der Well-Lotion behandelt. Während der Einwirkungszeit wurde nach ca. 15 Minuten erneut Well-Lotion auf das Haar gegeben. Nach 30 Minuten Einwirkzeit wurde mit Wasser (pH=7) gespült. Danach wurde das verformte Haar mit Ausnahme der Versuchsreihe 3 mit der Fixierlösung (2.1 bzw. 2.2) behandelt. Dann wurde das Haar 20 Minuten mit einer auf pH=7,5 eingestellten Pufferlösung (bestehend aus 2,2 Gew.-% KH₂PO₄, 14,6 Gew.-% K₂HPO₄ und Wasser) mit einer Temperatur von 30°C gewässert. Nach dem Trocknen wurde das Haar von den Wicklern befreit und die Kämmbarkeit und Weichheit (Avivage) des Haars benotet (1=sehr gut, 5=schlecht).

| **Versuchsreihe** | **1** | **2** | **3** | **4V** | **5V** |
|---|---|---|---|---|---|
| Well-Lotion | 1.2 | 1.3 | 1.1 | 1.2 | 1.3 |
| Fixier-Lösung | 2,1 | 2,2 | --- | 2,1 | 2,2 |
| Wässerung | 20 Min. | 20 Min. | 20 Min. | ----- | ----- |
| Kämmbarkeit | 1,5 | 1 | 2 | 4 | 3 |
| Weichheit | 2 | 1 | 2 | 3 | 2 |

## Patentansprüche

1. Verfahren zur dauerhaften Verformung von Keratinfasern, bei welchem man die Faser vor und/oder nach einer mechanischen Verformung einer Sulfitolyse durch Behandlung mit einer wäßrigen Lösung eines Sulfit-Salzes mit einem pH-Wert von 5-8 unterwirft, **dadurch gekennzeichnet, daß** die Keratinfasern nach der Sulfitolyse über einen Zeitraum von wenigstens 10 Minuten mit Wasser oder einer wäßrigen Zubereitung eines Puffersalzes mit einem pH-Wert von 6-9 nachehandelt werden.

2. Verfahren nach Anspruch 1, **dadurch gekennzeichnet, daß** die Lösung zur Durchführung der Sulfitolyse 5-15 Gew.-% Ammoniumhydrogensulfit enthält.

3. Verfahren nach Anspruch 1 oder 2, **dadurch gekennzeichnet, daß** die Lösung zur Durchführung der Sulfitolyse 1-5 Gew.-% Monoethanolamin enthält.

4. Verfahren nach einem der Ansprüche 1 bis 3, **dadurch gekennzeichnet, daß** die Lösung zur Durchführung der Sulfitolyse 5-20 Gew.-% 1,2-Propylenglycol enthält.

5. Verfahren nach einem der Ansprüche 1 bis 4, **dadurch gekennzeichnet, daß** die Lösung zur Durchführung der Sulfitolyse 1-10 Gew.-% Harnstoff enthält.

6. Verfahren nach einem der Ansprüche 1 bis 5 **dadurch gekennzeichnet, daß** vor der Nachbehandlung eine oxidative Fixierung der Keratinfasern durchgeführt wird.

## Claims

1. A process for the permanent deformation of keratin fibers in which, before and/after mechanical deformation, the fibers are subjected to sulfitolysis by treatment with an aqueous solution of a sulfite salt with a pH value of 5-8, **characterized in that**, after the sulfitolysis, the keratin fibers are aftertreated for at least 10 minutes with water or with an aqueous preparation of a buffer salt having a pH of 6 to 9.

2. A process as claimed in claim 1, **characterized in that** the solution for carrying out the sulfitolysis contains 5 to 15% by weight of ammonium hydrogen sulfite.

3. A process as claimed in claim 1 or 2, **characterized in that** the solution for carrying out the sulfitolysis contains 1 to 5% by weight of monoethanolamine.

4. A process as claimed in any of claims 1 to 3, **characterized in that** the solution for carrying out the sulfitolysis contains 5 to 20% by weight of 1,2-propylene glycol.

5. A process as claimed in any of claims 1 to 4, **characterized in that** the solution for carrying out the sulfitolysis contains 1 to 10% by weight of urea.

6. A process as claimed in any of claims 1 to 5, **characterized in that** the keratin fibers are oxidatively fixed before the aftertreatment.

## Revendications

1. Procédé pour la déformation permanente des fibres kératiniques, dans lequel on soumet les fibres, avant et/ou après une déformation mécanique, à une sulfitolyse par traitement avec une solution aqueuse d'un sel de sulfite présentant un pH de 5 à 8, **caractérisé en ce qu'**on procède à un post-traitement des fibres kératiniques, après la sulfitolyse, pendant une durée d'au moins 10 minutes, avec de l'eau ou une préparation aqueuse d'un sel tampon présentant un pH allant de 6 à 9.

2. Procédé selon la revendication 1, **caractérisé en ce que** la solution pour la réalisation de la sulfitolyse contient de 5 à 15 % en poids d'hydrogénosulfite d'ammonium.

3. Procédé selon la revendication 1 ou 2, **caractérisé en ce que** la solution pour la réalisation de la sulfitolyse contient de 1 à 5 % en poids de monoéthanolamine.

4. Procédé selon l'une des revendications 1 à 3, **caractérisé en ce que** la solution pour la réalisation de la sulfitolyse contient de 5 à 20 % en poids de 1,2-propylèneglycol.

5. Procédé selon l'une des revendications 1 à 4, **caractérisé en ce que** la solution pour la réalisation de la sulfitolyse contient de 1 à 10 % en poids d'urée.

6. Procédé selon l'une des revendications 1 à 5, **caractérisé en ce qu'**avant le post-traitement, on procède à une fixation par oxydation des fibres kératiniques.
